# EUROPEAN PATENT SPECIFICATION

(11) **EP 0 562 575 B1**
(45) Date of publication and mention of the grant of the patent: **16.09.1998**
(21) Application number: 93104869.8
(22) Date of filing: 24.03.1993
(51) Int. Cl.: C07D 251/46, A01N 47/36, C07C 311/39

(54) **N-substituted-3-(substituted hydrazino)-benzenesulfonamide derivative, preparation process thereof, and herbicidal compositions**
N-substituiertes-3-(substituiertes Hydrazino)-Benzolsulfonamidderivat, Verfahren zur Herstellung und herbizide Zusammensetzungen
Dérivé de benzenesulfonamide N-substitué-3-(hydrazino substitué), procédé pour la préparation et compositions herbicides

(30) Priority: 26.03.1992 JP 98597/92
(43) Date of publication of application: 29.09.1993
(73) Proprietor: KUREHA KAGAKU KOGYO KABUSHIKI KAISHA, Chuo-ku Tokyo 103 (JP)
(72) Inventor: Kanda, Yoichi, Iwaki-shi, Fukushima-ken 974 (JP); Arahira, Masato, Iwaki-shi, Fukushima-ken 974 (JP); Ito, Yoshiharu, Iwaki-shi, Fukushima-ken 974 (JP)
(74) Representative: Kraus, Walter, Dr.

(56) References cited:
- EP-A- 0 382 436
- EP-A- 0 382 437
- EP-A- 0 384 602

## Description

### BACKGROUND OF THE INVENTION

### Field of the Invention:

The present invention relates to N-substituted-3-(substituted hydrazino)benzenesulfonamide derivatives, a preparation process thereof, and herbicidal compositions containing the derivatives as active ingredients.

### Description of the Related Arts:

Numerous compounds have heretofore been proposed as herbicides. Regarding N-substituted benzenesulfonamide derivatives, a variety of compounds has also been reported as herbicides. For example, EP-A-116 518 and Japanese Patent Application Laid-Open (Kokai) No. 129276/1987 disclose a wide variety of compounds, of these N-substituted-3-substituted benzenesulfonamide derivatives to which -NHC(CH₃)=CHCOCH₃, -NHC(CH₃)=CHCOOCH_{3,} -NHCH₃, -OCH₂CF₃, or -SCH₂CF is bonded in the 3-position of the benzene nucleus of the benzenesulfonamide and to which a pyridine ring, or 1,3,5-triazine ring is bonded to the nitrogen atom, being disclosed therein. Furthermore, EP/A/382437 discloses N-substituted-3-substituted benzenesulfonamide derivatives to which -NHN=CHCF₃, -NHN=CHCOOH, or -NHN=CHCCl=CClCOOH is bonded in the 3-position of the benzene nucleus of the benzenesulfonamide and to which a pyridine ring, or 1,3,5-triazine ring is bonded to the nitrogen atom.

There has conventionally been a strong demand for herbicides capable of exhibiting reliable herbicidal activity even at such a low application dosage so that the amount present in the environment is reduced, herbicide capable of exhibiting selectivity between crops and weeds irrespective of variations in environmental conditions, herbicides which do not cause crop injury to the second crop in double cropping, etc.

The present invention has been completed with a view towards meeting such a demand.

An object of the present invention is, therefore, to provide novel compounds exhibiting excellent herbicidal effects, preparation process thereof, and intermediates from the process.

### SUMMARY OF THE INVENTION

The present inventors have discovered that compounds yet unreported in any publication and having a chemical structure different from the compounds disclosed in EP-A-116 518 and Japanese Patent Application Laid-Open (Kokai) No. 129276/1987 have excellent activities, resulting in the present invention.

The present invention has the following constructional characteristics:

A first aspect of the present invention relates to an N-substituted-3-(substituted hydrazino)benzene-sulfonamide derivative represented by the following formula (I): 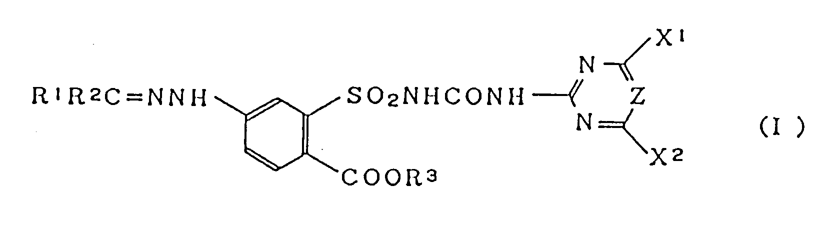 wherein R¹ is C₁-C₄ alkyl, monochloromethyl, C₃-C₄ alkenyl, C₁-C₄ alkoxymethyl, C₁-C₄ alkoxycarbonyl, C₁-C₄ alkylthioethyl, pyridyl, thienyl, thenyl, tetrahydrofurfuryl, morpholino or phenyl which is unsubstituted or substituted with chlorine or C₁-C₄ alkyl; R² is hydrogen or C₁-C₄ alkyl; R³ is C₁-C₄ alkyl; X¹ is C₁-C₃ alkyl, C₁-C₃ alkoxy, or chlorine; X² is C₁-C₃ alkyl or C₁-C₃ alkoxy; and Z is N or CH.

A second aspect of the present invention relates to a process for preparation of an N-substituted-3-(substituted hydrazino)benzenesulfonamide derivative represented by the foregoing formula (I) according to the following reaction formula. The process comprises reacting 2-amino-4,6-di-substituted-1,3,5-triazine or - pyrimidine with diphenyl carbonate to form a phenyl carbamate derivative represented by the formula (III), and further reacting the resulting product with 3-(substituted hydrazino)benzenesulfonamide derivative represented by the formula (II) without any intermediate isolation. 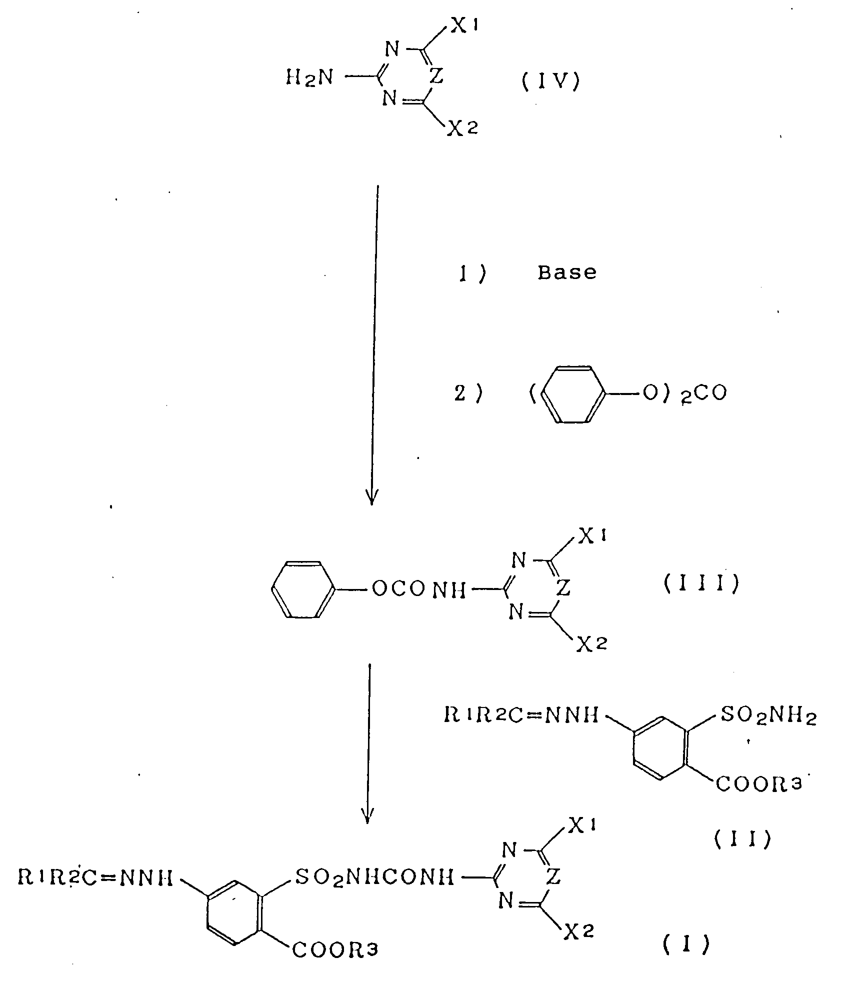 wherein R¹, R², R³, X¹, X², and Z have the same meanings as defined above.

A third aspect of the present invention is directed to a 3-(substituted hydrazino)benzenesulfonamide derivative represented by the formula (II) which is an intermediate for producing the above-mentioned N-substituted-3-(substituted hydrazino)benzene-sulfonamide derivative. 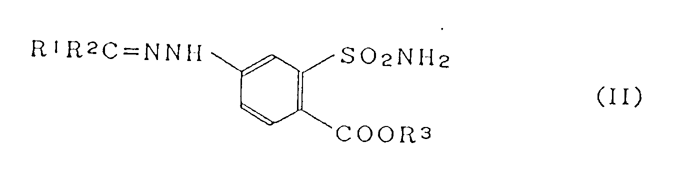 wherein R¹, R², and R³, have the same meanings as defined above.

A fourth aspect of the present invention is provided for a herbicidal compound, which contains the above-mentioned N-substituted-3-(substituted hydrazino)benzenesulfonamide derivative represented by the formula (I) as an active ingredient in the herbicidally effective amount.

### DETAILED DESCRIPTION OF THE INVENTION

The present invention will now be described in detail.

In the following description, the symbols R¹, R², R³, X¹, X², and Z have the same meanings as defined above and, thus, their descriptions are omitted.

Specific examples of N-substituted-3-(substituted hydrazino)benzenesulfonamide derivatives represented by the formula (I) are shown in Table 1.

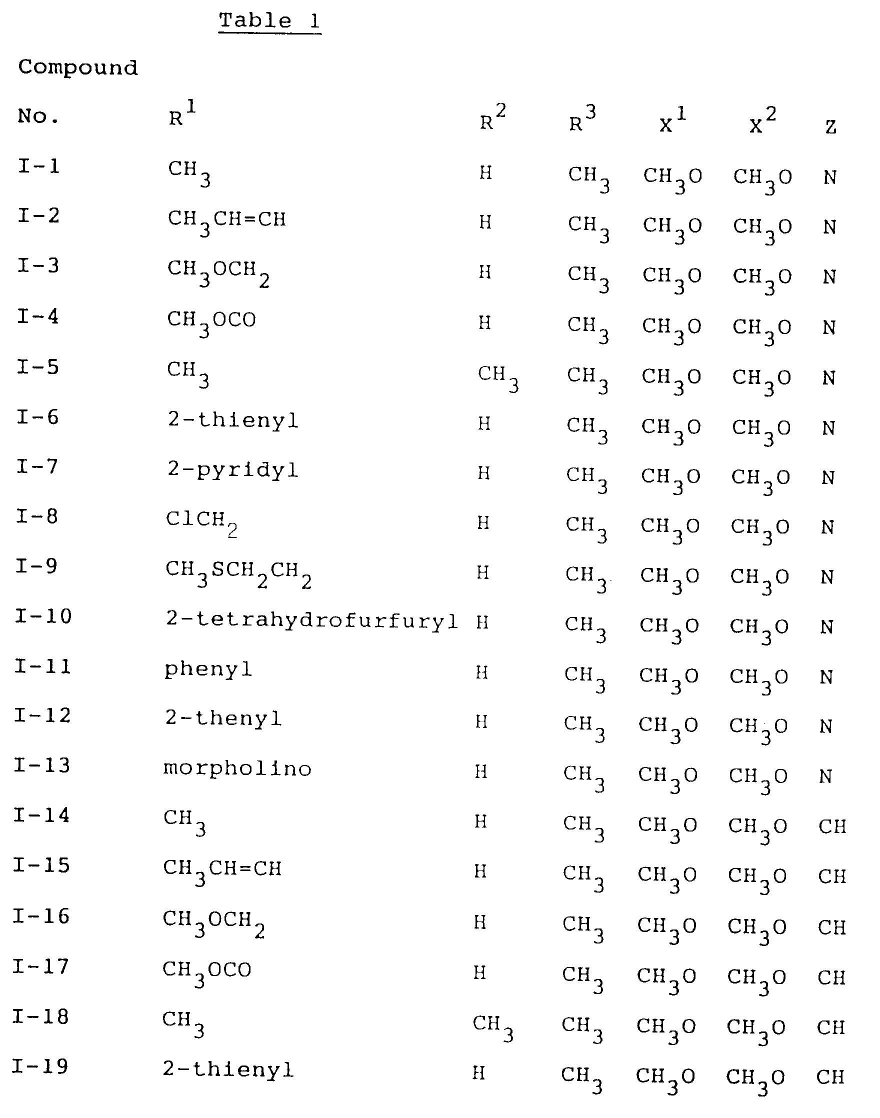

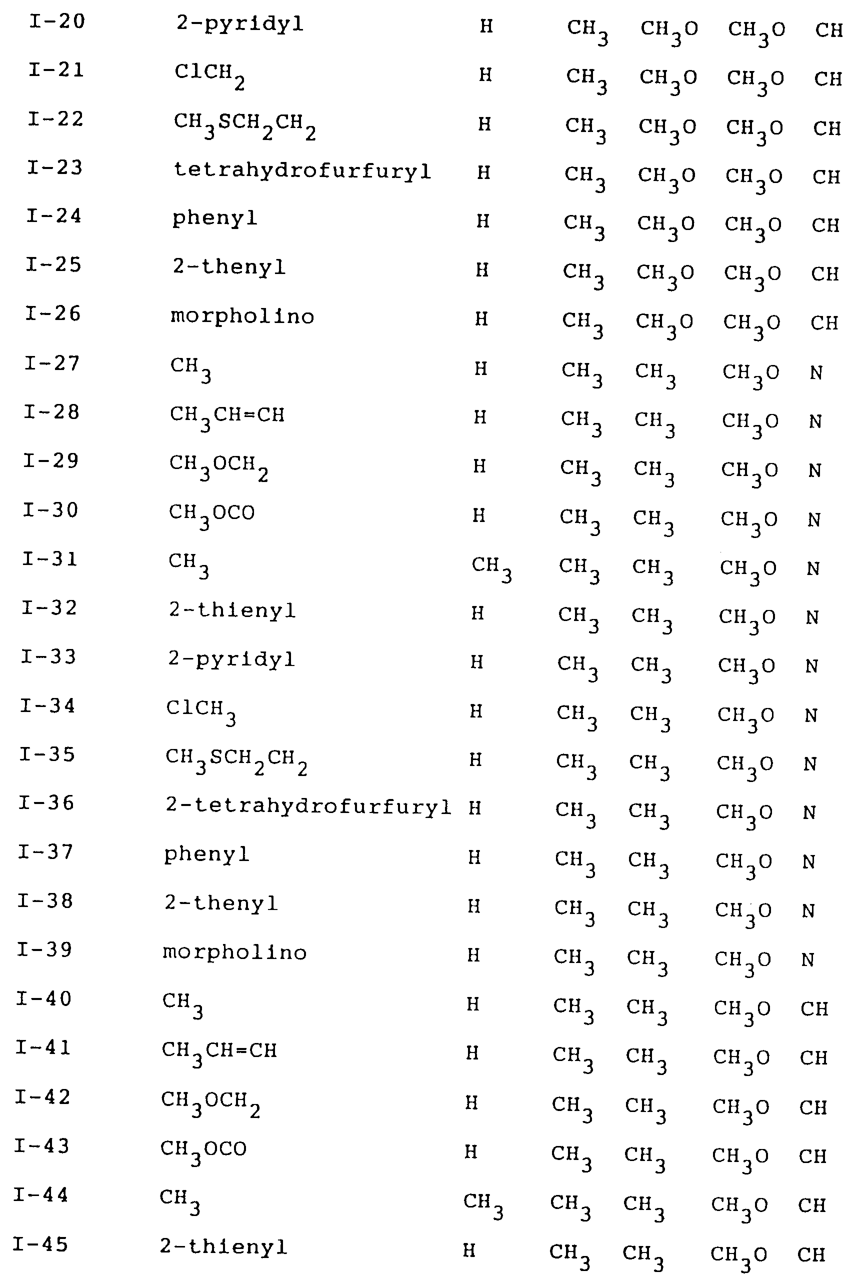

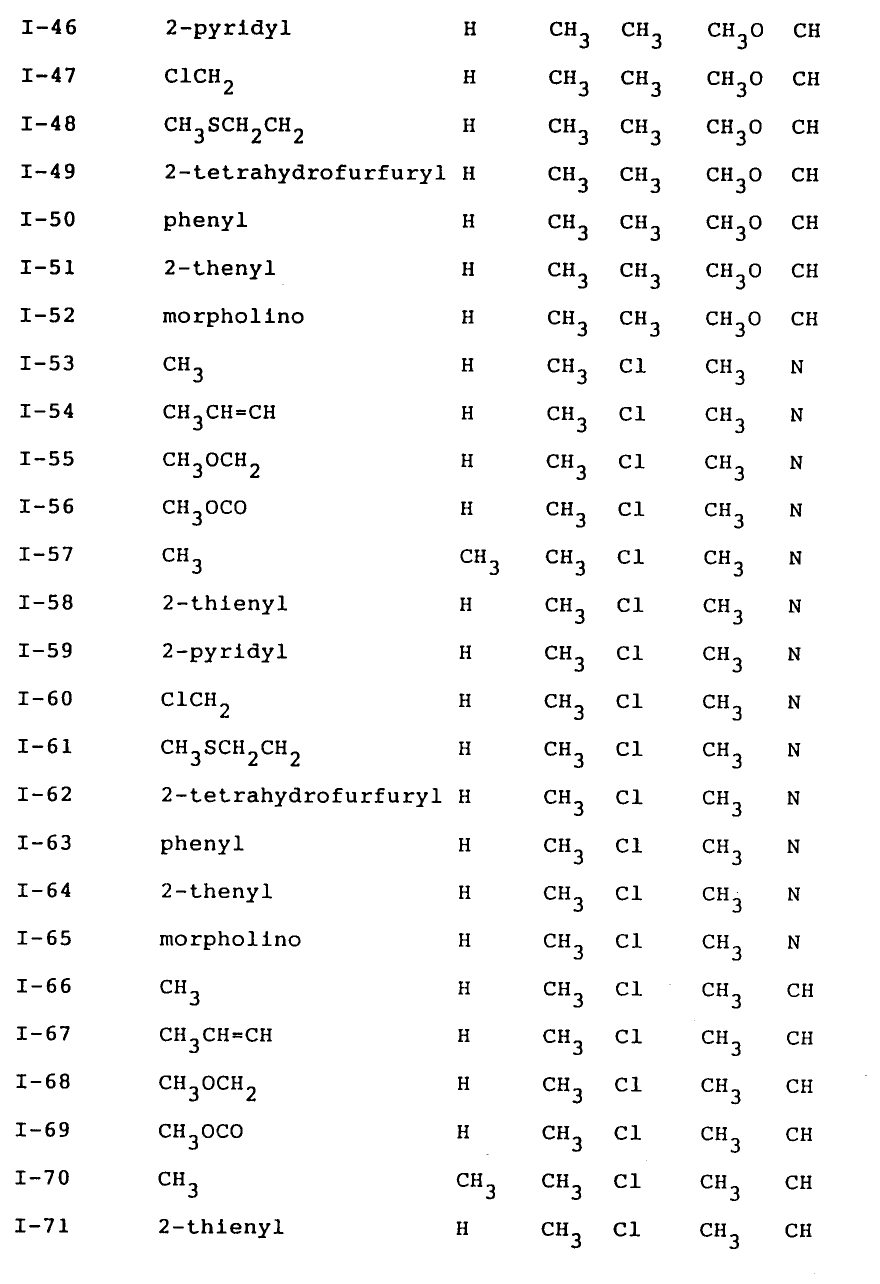

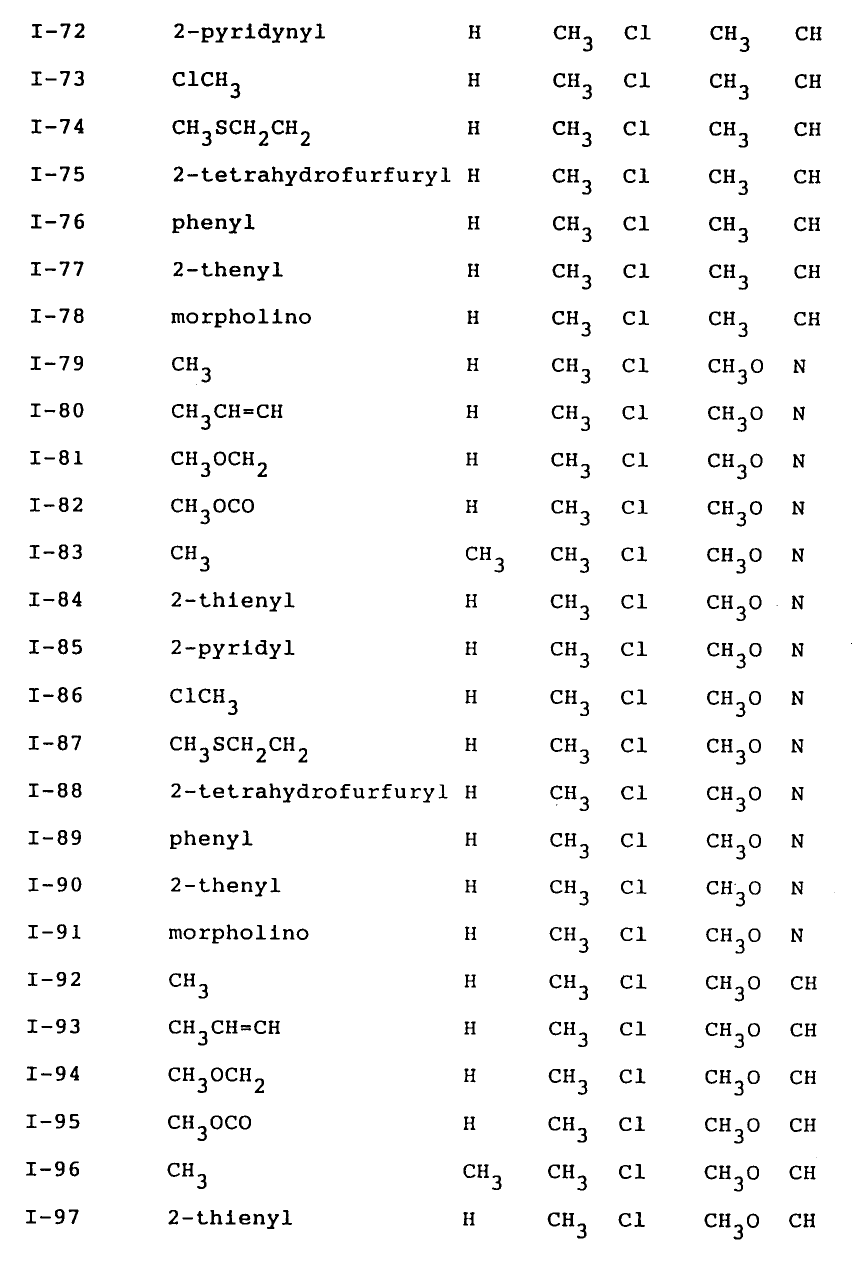

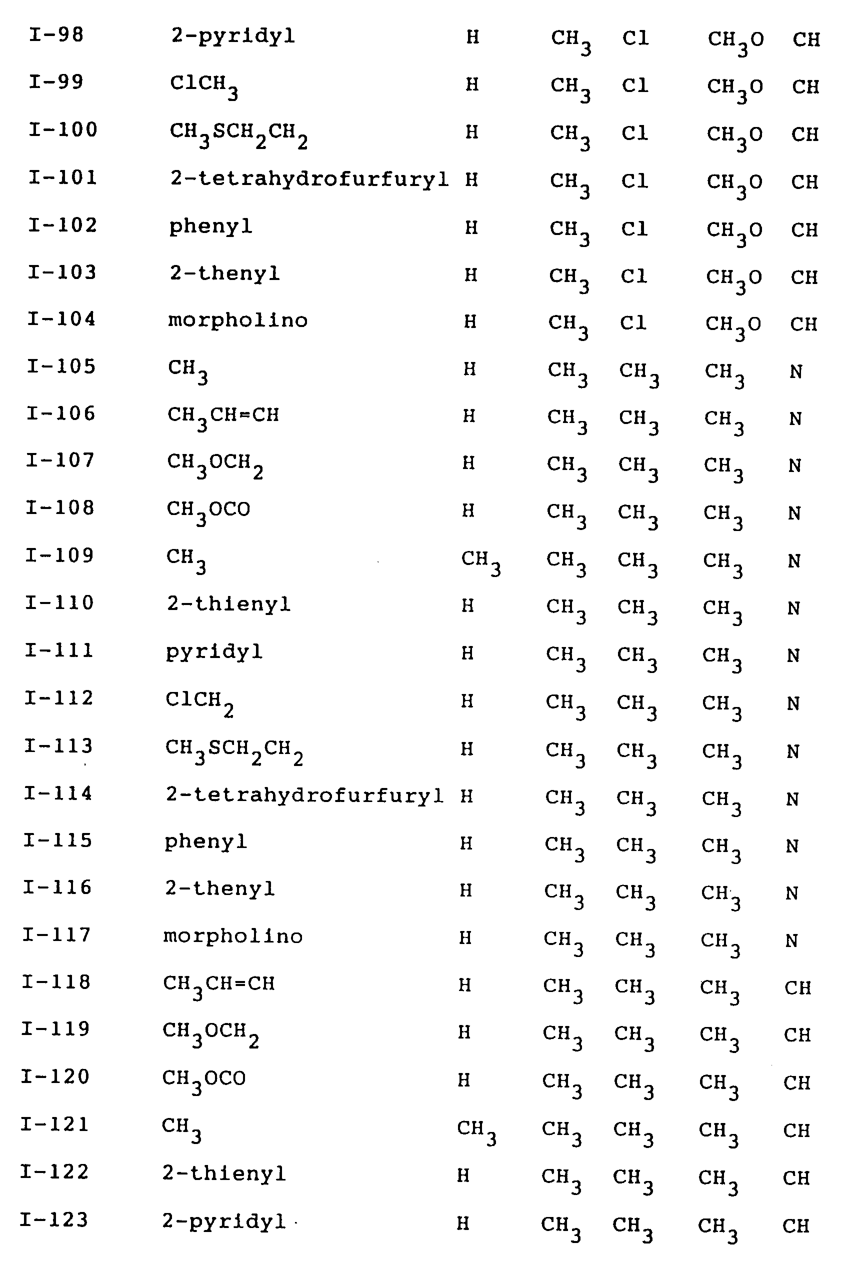

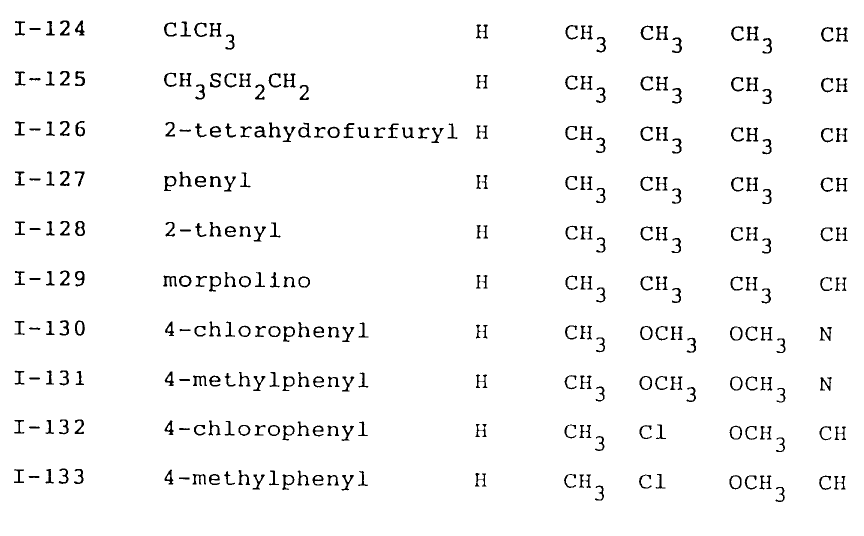

According to the following reaction formula, the N-substituted-3-(substituted hydrazino)benzenesulfonamide derivatives represented by the formula (I) can be synthesized by a 3- (substituted hydrazino) benzenesulfonamide derivative represented by the formula (II) with a phenyl carbamate derivative represented by the formula (III) in an organic solvent in the presence of a base.

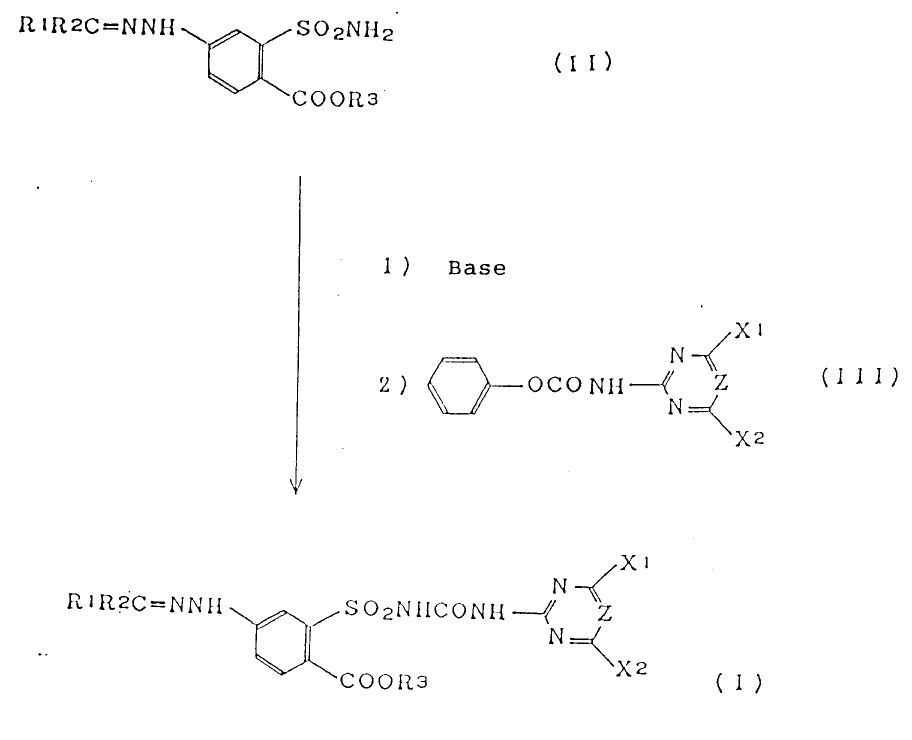

According the reaction formula just mentioned, the derivative can be synthesized by the following processes (A) or (B).

### Process (A):

A process in which a phenyl carbamate derivative represented by the formula (III) is synthesized and isolated by the process described in EP No. 238,070, followed by reacting it with a 3-(substituted hydrazino) benzenesulfonamide represented by the formula (II).

### Process (B):

A process in which a 2-amino-4,6-di-substituted-1,3,5-triazine or -pyrimidine represented by the formula (IV) is added to an organic solvent, a base is then added thereto, the mixture is stirred for a several period of time, after which, diphenyl carbonate is added, and then, without isolating a phenyl carbamate derivative represented by the formula (III), the 3-(substituted hydrazino) benzenesulfonamide represented by the formula (II) is further reacted.

In the event where the N-substituted-3-(substituted hydrazino) benzenesulfonamide derivative represented by the formula (I), wherein Z is N, is synthesized, preference is given to process B.

In the reactions according to process A and process B, dimethylacetamide, N-methylpyrrolidone, acetonitrile, and similar solvents may be used as the organic solvent.

Sodium hydride, diazabicyclooctane, diazabicyclononene, etc. may be used as a base. In process B, sodium hydride is proven to be preferable. The reaction is carried out at a temperature in the range of -20 to 100°C, preferably 0 to 50°C, for a period of 0.5 to 24 hours, preferably 0.5 to 6 hours.

After the reaction has been completed, the reaction mixture is added to a diluted aqueous solution of hydrochloric acid, the resulting precipitate is filtered off, and dried in air, after which the product is purified with chromatography or by washing. This can give the intended N-substituted-3-(substituted hydrazino)benzenesulfonamide derivative represented by the formula (I).

In the reactions mentioned above, the 3-(substituted hydrazino)benzenesulfonamide represented by the formula (II), which is an intermediate serving as a starting material, can be synthesized using a 3-aminobenzenesulfonamide derivative represented by the formula (V) as a starting substance:

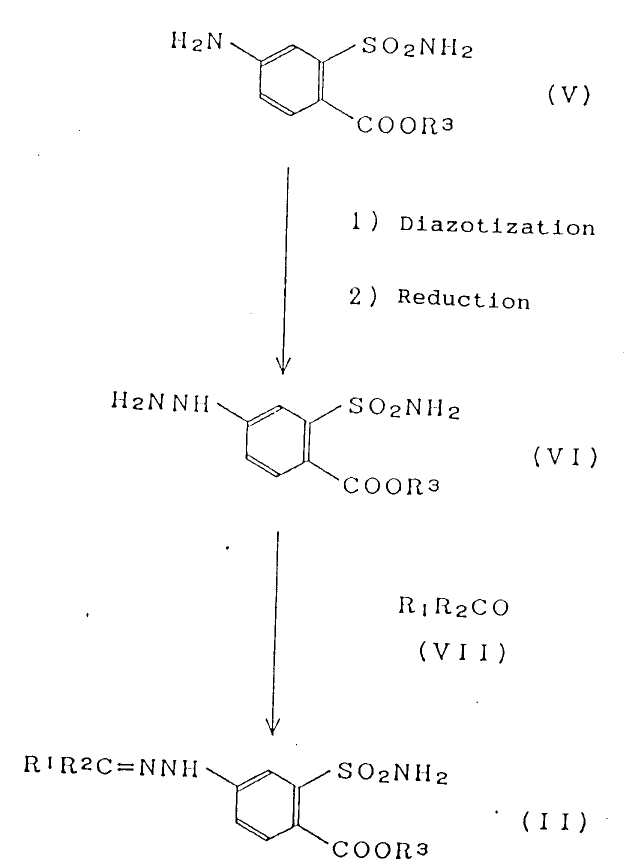

The synthesis of the compound represented by the formula (II) according to the foregoing reaction formula can be carried out as follows: The amino group of the 3-aminobenzenesulfonamide derivative represented by the formula (V) is diazotiated, and reduced to obtain a 3-hydrazinobenzenesulfonamide derivative represented by the formula (VI), which is then, together with an aldehyde or ketone, stirred in methanol or dioxane at a temperature of 30 to 120°C, preferably 70 to 90°C, over 0.5 to 8 hours. After the reaction has been completed, the reaction product is filtered off, or alternatively the reaction product is dried in vacuo to solid and, if necessary, purified by washing, whereby the compound represented by the formula (II) can be obtained with a good purity. The 3-aminobenzenesulfonamide derivative represented by the formula (V), which is used in the above-mentioned reaction, can here be obtained according to the following reaction formula: The 4-nitro-2-(aminosulfonyl)benzoic acid represented by the formula (VIII) is esterified with an alcohol represented by the formula (IX), and the nitro group of the resulting benzoic ester derivative represented by the formula (X) is reduced with SnCl₂ in an alcohol represented by the formula (IX) containing 35% hydrochloric acid.

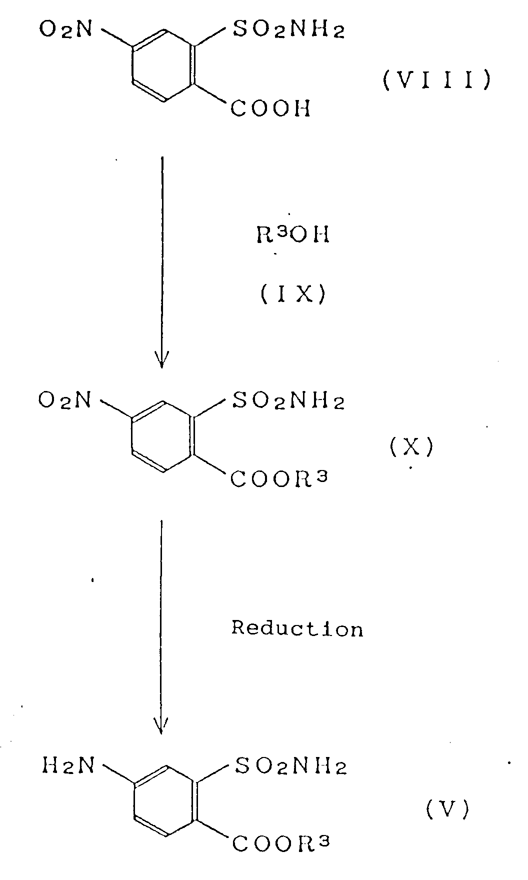

Specific examples of the compound represented by the formula (II), which can be used as the above-mentioned intermediate, are listed in Table 2.

**Table 2**

| Compound No. | R¹ | R² | R³ |
|---|---|---|---|
| II-1 | CH₃ | H | CH₃ |
| II-2 | CH₃CH=CH | H | CH₃ |
| II-3 | CH₃OCH₂ | H | CH₃ |
| II-4 | CH₃OCO | H | CH₃ |
| II-5 | CH₃ | CH₃ | CH₃ |
| II-6 | 2-thienyl | H | CH₃ |
| II-7 | 2-pyridyl | H | CH₃ |
| II-8 | ClCH₂ | H | CH₃ |
| II-9 | CH₃SCH₂CH₂ | H | CH₃ |
| II-10 | 2-tetrahydrofurfuryl | H | CH₃ |
| II-11 | phenyl | H | CH₃ |
| II-12 | 2-thenyl | H | CH₃ |
| II-13 | morpholino | H | CH₃ |
| II-14 | 4-chlorophenyl | H | CH₃ |
| II-15 | 4-methylphenyl | H | CH₃ |

The N-substituted-3-(substituted hydrazino) benzenesulfonamide derivative of the formula (I) according to the present invention (hereinafter referred to as "the compound of the present invention") exhibits a certain herbicidal effect in a low dosage, and has a selectivity between crops and weeds. Accordingly, a herbicidal composition comprising this compound as an active ingredient is suitable, e.g., for controlling monocotyledous and dicotyledous weeds in important crops, such as wheat, rice, corn, soybean, cotton, sugar beet, potato, and tomato, before or after germination.

Examples of dicotyledon weeds which can be prevented by the herbicidal composition of the present invention include weeds belonging to Amarnthus, Bindens, Stellaria, Abutilon, Convolvulus, Matricaria, Galium, Lindernia, and the like.

Examples of monocotyledon weeds which can be prevented by the herbicidal composition of the present invention include weeds belonging to Echinochloa, Setaria, Digitaria, Avena, Cyperus, Alisma, Monochoria, and the like.

The herbicidal composition according to the present invention may be applied in areas such as farming areas, inclusive of farms, paddy fields, and orchards, as well as non-farming areas, inclusive of grounds and industrial sites.

The compound of the present invention can be utilized as is, but it is generally used in various forms of preparations, such as dust, wettable powder, granule, emulsion, etc., together with a preparation adjuvant.

In this case, a preparation is prepared so that one or more compounds of the present invention are contained in amounts of 0.1 to 95% by weight, preferably 0.5 to 90% by weight, more preferably 2 to 70% by weight.

Examples of carriers, diluents, and surfactant, which can be used as the preparation adjuvant, include talc, kaolin, bentonite, diatom earth, white carbon, clay, and the like. As liquid diluents, water, xylene, toluene, chlorobenzene, cyclohexane,cyclohexanone, dimethylsulfoxide, dimethylformamide, alcohols, and the like can be exemplified.

Surfactants are preferably used depending upon their effects. Examples of emulsifiers are polyoxyethylene alkylaryl ethers, polyoxyethylene sorbitan monolaurate, etc.; examples of dispersants are lignin sulfonates, dibutylnaphthalene sulfonate, etc.; and examples of wetting agents are alkylsulfonates, alkylphenyl sulfonates, etc.

The above-mentioned preparation can be roughly divided into one which is used as is and one which is used by being diluted with a diluent such as water. In the case where the concentration of the compound of the present invention preferably ranges from the 0.01 to 1.0%. The amount of the compound of the present invention used is in the range of 0.01 kg to 10 kg, preferably 0.05 to 5 kg, per 1 ha.

Since the concentrations and amounts used vary with the form of preparation, the period of usage, the manners of usage, the sites of the usage, the intended crops, and other factors, these values can, of course, be changed in spite of the above ranges. Moreover, the compound of the present invention can be used in combination with other active ingredients, such as fungicides, bactericides, miticides, and herbicides.

The above-mentioned N-substituted-3-(substituted hydrazino)benzenesulfonamide derivative of the formula (I) is a compound which has not yet been described in literature, exhibits a certain herbicidial effect in a low dosage, and has a selectivity between crops and weeds. Accordingly, the herbicidial composition comprising this compound as an active ingredient is suitable, e.g., for controlling monocotyledous and dicotyledous weeds in important crops, such as wheat, rice, corn, soybean, cotton, sugar beet, potato, and tomato, before or after germination. The composition can be applied in areas such as farming areas, inclusive of farms, paddy fields, and orchards, as well as non-farming areas, inclusive of grounds and industrial sites.

### EXAMPLES

The present invention will now be described in detail by referring to Synthesis Examples of the N-substituted-3-(substituted hydrazino)benzenesulfonamide, which is the compound according to the present invention, Preparation Examples and Test Examples thereof. Various modifications and alternations can be made as long as they deviate from the scope of the present invention and, thus, the present invention is not intended to be limited to these Synthesis Examples, Preparation Examples, and Test Examples.

### Synthesis Example 1

### Synthesis of 4-[(Ethylidene)hydrazino]-2[[[(4,6-dimethoxy-1,3,5-triazine-2-yl)amino]carbonyl]amino]sulfonyl]benzoic acid methyl ester (Compound No.I-1)

In 18 ml of dry dimethylacetamide were suspended 1.82 g (11.7 mmol) of 2-amino-4,6-dimethoxy-1,3,5-triazine, and the suspension was stirred. To this was added 0.46 g (11.7 mmol) of 60% strength sodium hydride. After bubbling was over, the reaction mixture became clear solution. Thereafter, a solution of 2.5 g (11.7 mmol) diphenyl carbonate in 6 ml of dimethylacetamide was added dropwise. After stirring for 10 minutes, a solution of 2.87 g (10.6 mmol) of methyl 2-(aminosulfonyl)-4-[(ethylidene) hydrazio]benzoate in 12 ml of dimethylacetamide was added to this solution under water-cooling. The reaction solution turned brown. The reaction solution was stirred for 1 hour and 50 minutes. Subsequently, the reaction solution was poured into 170 ml of ice-water containing 1.2 ml of 35% hydrochloric acid.

Thereafter, the precipitated solid was filtered off, washed with water, and then dried in air. The crude product was purified with column chromatography (eluent: ethyl acetate/n-hexane =1/1) on silica gel (Wako-gel C-300), and washed with petroleum ether. This gave 1 g of a white solid (yield 20.4%) having a melting point of 130°C (decomposition). The physical properties of the product are shown in Table 3.

### Synthesis Examples 2 to 7

Other N-substituted-3-(substituted hydrazino) benzenesulfonamide derivatives listed in Table 1 can be synthesized as in Synthesis Example 1. Their physical properties are shown in Table 3.

In Table 3, and Table 4, which will be described later on, the abbreviations for NMR have the following meanings:
s (singlet),d (doublet), t (triplet), q (quadruplet), m (multiplet), dd (double doublet), br (broad), bs (broad singlet).

**Table 3**

| Compound No. | M.P (or decomp) (°C) | IR (KBr) cm-1 | NMR Solvent δ |
|---|---|---|---|
| I-1 | 130 decomp. | 3400-2980, 1728, 1068, 1498, 1358, 1272, 1130 | CDCl3 1.08(3H,d,J=7Hz) 3.76(3H,s) 4.0(6H,s) 6.9-8.3(6H,m) |
| I-2 | 120 decomp. | 3400-2980, 1730, 1606, 1498, 1382, 1274, 1116 | CDCl3 1.8(3H,J=5Hz) 3.8(3H,s) 4.0(6H,s) 6.0-8.78(8H,m) |
| I-3 | 120 decomp. | 3380-2900, 1732, 1606, 1498, 1454, 1382, 1274, 820, 586 | CDCl3 3.35(3H,s) 3.8(3H,s) 4.0(6H,s) 4.06(2H,d,J=5Hz) 7-8.67(6H,m) 12.08(1H,bs) |
| I-4 | 140 decomp. | 3350-2980, 1730, 1576, 1498, 1454, 1382, 1278, 1146, 820 | CDCl3 3.8(6H,s) 4.0(6H,s) 7.18-8.85(5H,m) 9.98(1H,bs) 12.1(1H,bs) |
| I-5 | 184 decomp. | 3375-2990, 1735, 1620, 1580, 1390, 1370, 1305 | d6-DMSO 1.94(3H,s) 2.0(3H,s) 3.76(3H,s) 4.0(6H,s) 7.3(1H,dd,J=2Hz,8Hz) 7.7(1H,d,J=8Hz) 7.9(1H,d,J=2Hz) 9.63(1H,s) 10.9(1H,s) 12.1(1H,s) |
| 1-6 | 176 decomp. | 3240, 3111, 1720, 1600, 1565, 1461, 1380, 1350, 1275 | d6-DMSO 3.8(3H,s) 4.0(6H,s) 7.25-8.2(7H,m) 10.9(1H,s) 11(1H,s) 12(1H,s) |
| I-7 | 130 decomp. | 3300-2950, 1728, 1604, 1498, 1454, 1358, 1276, 1170, 1026, 890, 820 | CDCl3 3.8(3H,s) 4.0(6H,s) 7.3-9.1(10H,m) 11.6(1H,bs) |

### Synthesis Example 8

### Synthesis of 2-(aminosulfonyl)-4-[(ethylidene)hydrazino]benzoic acid methyl ester (compound No. II-1)

### (1) Synthesis of 2-(aminosulfonyl)-4-hydrazinobenzoic acid methyl ester (compound of the formula (VI) wherein R³ = CH₃)

To a mixture of 6 ml of 35% hydrochloric acid and 6 ml of water were added 3 g (13 mmol) of 4-amino-2-(aminosulfonyl)benzoic acid methyl ester (compound of the formula (V) wherein R³ = CH₃), and the mixture was stirred at room temperature for 5 minutes. This was then allowed to cool by means of ice-water, stirred, and then diazotiated by the addition of 0.95 g (13.7 mmol) of sodium nitrite dissolved in 3 ml of water.

A solution of 6.78 g (68.5 mmol) of stannous chloride dissolved in 6.52 ml of 35% hydrochloric acid was added to the previously prepared diazonium compound under ice-cooling with stirring.

After stirring for 20 minutes, the mixture was left standing in a refrigerator over a period of 15 hours. The reaction mixture was then transferred into a 3-liter volume beaker, and extracted twice with each 300 ml of ethyl acetate. The extract was dried over sodium sulfate, and the extracting solvent was distilled off to obtain 2.76 g (yield 86%) of a pale yellow solid having a melting point of 168-170°C.

### (2) Synthesis of 2-(aminosulfonyl)-4-[(ethylidene)hydrazino]benzoic acid methyl ester

To a solution of 0.5 g (2 mmol) of the compound synthesized under the above section (1) (compound of the formula (V) wherein R³ = CH₃) dissolved in methanol was added 0.3 g (6.8 mmol) of acetaldehyde, and the mixture was stirred at 70°C for 3 hours. Subsequently, methanol was removed from the reaction mixture, and the residue was filtered off, followed by washing with a small amount of methanol, and drying in air to give 0.316 g (yield 57.30%) of a yellow solid. The physical properties of the product are listed in Table 4.

### Synthesis Examples 9 to 14

Other 3- (substituted hydrazino)benzenesulfonamide derivatives listed in Table 4 can be prepared as in Synthesis Example 8. Their physical properties are listed in Table 4.

**Table 4**

| Compound No. | State | M.P. or Decomp. °C | IR (KBr) cm -¹ | NMR Solvent δ |
|---|---|---|---|---|
| II-1 | Yellow Solid | 190 192 | 3324, 1696, 1606, 1438, 1340, 1296, 1172, 774 | d₆-DMSO: 1.9 (3H,d,J=6Hz), 3.8 (3H,s) 6.7 (1H, q,J = 6Hz). 6.9 -7.9 (5H,m) 9.8- 10.5 (1H,br) |
| II-2 | Yellow Solid | 158 160 | 3330, 1696, 1608, 1438, 1336, 1276, 1172, 1120 | d₆-DMSO:1.8 (3H, d ,J = 5Hz), 3.75 (3H,s) 5.93-7.7(8H,m) 10.75 (1H,br) |
| II-3 | Yellow Solid | 171 172 | 3372, 3268, 1694, 1600, 1438, 1336, 1274, 1162, 774, 702 | d₆-DMSO: 3.3(3H,s) 3.81 (3H,s) 4.0 (2H,d,J=5Hz) 7.0-7.8 (6H,m) 10,8 (1H,bs) |
| II-4 | Yellow Solid | 226 227 decomp. | 3300, 1712, 1610, 1576, 1438, 1292, 1164 | d₆-DMSO:3.73 (3H,s) 3.8 (3H,s) 7.2(2H,bs) 7.25-7.88 (4H,m) 11.8 (1H,s) |
| II-5 | Yellow Solid | 190 193 | 3360, 3290, 1710, 1620, 1310, 1130 | d₆-DMSO: 1.9 (3H,s) 1.95 (3H,s) 3.75 (3H,s) 7.0 (2H,s) 7.1 (1H,dd,J=2Hz,9Hz) 7.6 (1H,d,J=9) 7.68 (1H,d,J=2Hz) 9.46 (1H,s) |
| II-6 | Yellow Solid | 176 decomp. | 3400, 3325, 1720, 1625, 1450, 1345, 1290, 1180, 1140, 790 | d₆-DMSO: 3.8 (3H,s) 7.2 (2H,bs) 7.25-8.2 (7H,m) 11.25 (1H,bs) |
| II-7 | Yellow Solid | 230 decomp. | 3280, 1712, 1610, 1566, 1432, 1320, 1272, 1194, 1154, 1122, 776, 706 | d₆-DMSO: 3.83 (3H,s) 7.2(2H,bs) 7.26- 8.31(6H,m) 8.56 (1H,dd,J=2Hz, 5Hz) 8.9 (1H,d,J=2Hz) 11.25 (1H,bs) |

Preparation Examples and Test Examples will now be described, in which carriers (diluents) and auxiliaries, mixing ratio thereof, and active ingredients can be altered in a wide range.

### Preparation Example 1 (Wettable powder)

- 50 parts: of Compound (I-1),
- 5 parts: of lignin sulfonate,
- 3 parts: of alkylsulfonate, and
- 42 parts: of Diatom earth
are mixed, and ground to prepare a wettable powder, which is used with diluting with water.

### Preparation Example 2 (Emulsion)

- 25 parts: of Compound (I-3)
- 65 parts: of xylene, and
- 10 parts: of polyoxyethylene alkylaryl ether
are uniformly mixed to prepare an emulsion, which is used with diluting with water.

### Preparation Example 3 (Granule)

- 8 parts: of Compound (I-4),
- 40 parts: of bentonite,
- 45 parts: of clay, and
- 7 parts: of lignin sulfonic acid
are uniformly mixed intimately and after the addition of water, were kneaded and then formed into granules by an extruding granulator. They are then dried to provide a granular formulation, namely, a granule.

### Test Example 1

### Germination Test of seeds:

Two sheets of filter paper were placed in a superposed relation in each of Petri dishes having a diameter of 9 cm. Water suspensions of each test compound (concentrations of the active ingredient: 0.25 ppm and 2.5 ppm) were separately poured in an amount of 6ml per dish into the Petri dishes. Seeds of Amaranthus retroflexus, (Redroot pigweed), Bidens pilosa (Common blackjack), Matricaria chamomilla (Wild chamomile), Solanum nigrum (Black nightshade), Echinochloa crus-galli(Barnyard grass), Cyperus iria (Rice flatsedge) and Setaria viridis (Green foxtail) were placed at a rate of 10 seeds per dish in the Petri dishes. They were thereafter allowed to germinate in a constant-temperature chamber at 28 °C. Fourteen days later after placement in the Petri dishes, the degrees of germination and growth inhibition were observed visually. The observation results were ranked in accordance with the below-described 6-stage system. The results are summarized in Table 5.

### Growth inhibition rate;

- 0:: no inhibition
- 1:: less than 31%;
- 2:: 31 - 50%;
- 3:: 51 - 70%;
- 4:: 71 - 90%;
- 5:: 91 - 100%.

**Table 5**

| Compound No. | Concentration ppm | A.r. | B.p. | M.c. | S.n. | E.c. | C.i. | S.v. |
|---|---|---|---|---|---|---|---|---|
| I-1 | 0.25 | 3 | 3 | 2 | 2 | 2 | 2 | 3 |
| | 2.5 | 5 | 4 | 4 | 3 | 3 | 4 | 4 |
| I-2 | 0.25 | 3 | 3 | 1 | 3 | 2 | 2 | 2 |
| | 2.5 | 4 | 4 | 2 | 4 | 2 | 3 | 3 |
| I-3 | 0.25 | 2 | 3 | 2 | 2 | 2 | 4 | 3 |
| | 2.5 | 3 | 4 | 3 | 3 | 3 | 4 | 3 |
| I-4 | 0.25 | 2 | 2 | 1 | 1 | 1 | 2 | 3 |
| | 2.5 | 3 | 3 | 2 | 2 | 2 | 4 | 3 |
| I-5 | 0.25 | 1 | 3 | 1 | 2 | 4 | 3 | 1 |
| | 2.5 | 3 | 4 | 2 | 3 | 2 | 4 | 2 |
| I-6 | 0.25 | 2 | 3 | 2 | 1 | 1 | 2 | 1 |
| | 2.5 | 3 | 4 | 3 | 2 | 2 | 4 | 2 |
| I-7 | 0.25 | 3 | 2 | 3 | 2 | 2 | 2 | 2 |
| | 2.5 | 4 | 4 | 4 | 3 | 3 | 4 | 2 |
| Note: A.r. Amaranthus retroflexus B.p. Bidens pilosa M.c. Matricaria chamomilla S.n. Solanum nigrun E.c. Echnochloa crus-galli C.i. Cyperus iria S.v. Setaria viridis | | | | | | | | |

### Test Example 2

### Test on Herbicidal Activity by Foliar Application

Herbicidal solutions of each test compound, which had been prepared by dissolving at predetermined concentrations such as wettable powder of the test compound as that described in the above formulation example, and sprayed at dosages of 5 g/ha and 50 g/ha over foliar parts of Amaranthus retroflexus, (Redroot pigweed), Bidens pilosa (Common blackjack), Sinapis arvensis (Wild mustard), Stellaria media (Common chickweed), Cassia obtusifolia (Sicklepod), Solanum nigrum (Black nightshade), Abutilon theophrasti (Velvetleaf), Convolvulus arvensis (Field bindweed), Matricaria chamomilla (Wild chamomile), Setaria viridis (Green foxtail), Echinochloa frumentaceum (Barnyard grass), Avena fatua (Wild oat), and Digitaria adscendens (Henry crabgrass) which had been allowed to grow individually to 2 - 4 leaf stage in pots. Fourteen days later after spraying of the test compound, its herbicidal acrivity was evaluated in accordance with the below-described system. The results are summarized in Table 6.

### Ranking system:

### Herbicidal activity

- 0:: no effects
- 1:: less than 30% of total kill
- 2:: 31 - 50% of total kill
- 3:: 51 - 70% of total kill
- 4:: 71 - 90% of total kill
- 5:: 91 - 100% of total kill.

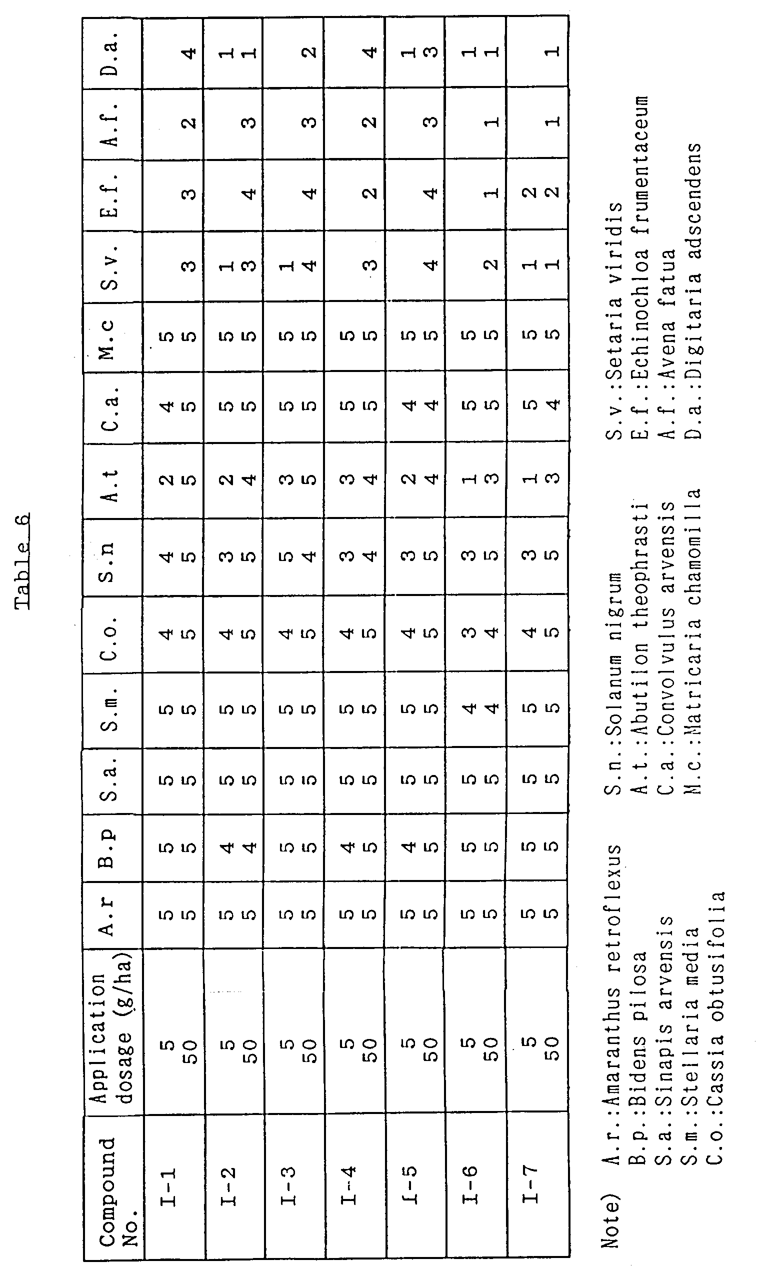

### Test Example 3

### Test for Selective Herbicidal Effect in Post-emergence by Treatment of Stalks and Leaves

Wheat and the weed were reared from seeds in the planters in a glasshouse. When the young plants had reached the 1 - to 2 - leaf stage, the wettable powders prepared in the same manner as Preparation Example 1 described above were diluted with water to a prescribed concentration and sprayed uniformly to the stalks arid leaves of the plants and on the soil surface in the planters by a spray gun so that the acrive ingredient would be applied at a rate of 5 g/ha and 50 g/ha. Then the planters were kept in the glasshouse. Twenty-one days after said treatment, the herbicidal effect of the compositions on the weeds and phytotoxicity of the crops were observed and evaluated according to the following ratings.

The results are shown in Table 7.

### Growth inhibition rate

- 0:: no inhibition
- 1:: less than 31%
- 2:: 31 - 50%
- 3:: 51 - 70%
- 4:: 71 - 90%
- 5:: 91 - 100%

**Table 7**

| Compound No. | Amount | Galium aparine | Wheat |
|---|---|---|---|
| I-1 | 5 | 5 | 0 |
| | 50 | 5 | 0 |
| I-2 | 5 | 5 | 0 |
| | 50 | 5 | 0 |
| I-3 | 5 | 5 | 0 |
| | 50 | 5 | 0 |
| I-4 | 5 | 5 | 0 |
| | 50 | 5 | 0 |
| I-5 | 5 | 5 | 0 |
| | 50 | 5 | 0 |
| I-6 | 5 | 5 | 0 |
| | 50 | 5 | 0 |
| I-7 | 5 | 5 | 0 |
| | 50 | 5 | 0 |

## Claims

1. An N-substituted-3-(substituted hydrazino)benzenesulfonamide derivative represented by the following formula (I): 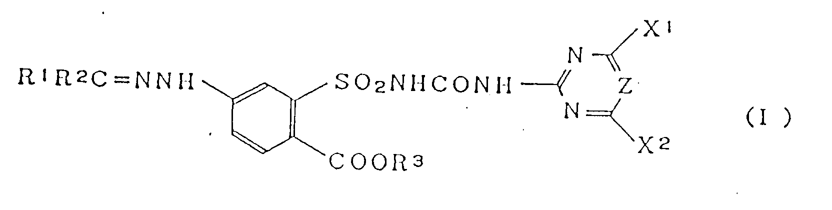 wherein R¹ is C₁-C₄ alkyl, monochloromethyl, C₃-C₄ alkenyl, C₁-C₄ alkoxymethyl, C₁-C₄ alkoxycarbonyl, C₁-C₄ alkylthioethyl, pyridyl, thienyl, thenyl, tetrahydrofurfuryl, morpholino or phenyl which is unsubstituted or substituted with chlorine or C₁-C₄ alkyl; R² is hydrogen or C₁-C₄ alkyl; R³ is C₁-C₄ alkyl; X¹ is C₁-C₃ alkyl, C₁-C₃ alkoxy, or chlorine; X² is C₁-C₃ alkyl or C₁-C₃ alkoxy; and Z is N or CH.

2. The N-substituted-3-(substituted hydrazino) benzenesulfonamide derivative as claimed in Claim 1, wherein Z in the general formula (I) is N.

3. The N-substituted-3-(substituted hydrazino) benzenesulfonamide derivative as claimed in Claim 1, wherein Z in the general formula (I) is CH.

4. A process for producing an N-substituted-3-(substituted hydrazino)benzenesulfonamide derivative represented by the formula (I): 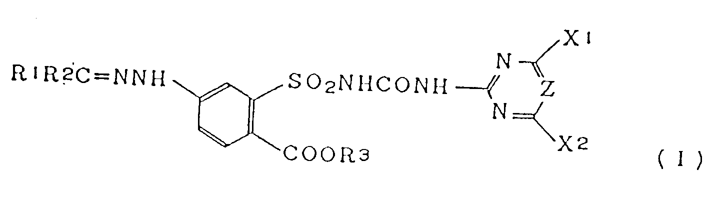 wherein R¹ is C₁-C₄ alkyl, monochloromethyl, C₃-C₄ alkenyl, C₁-C₄ alkoxymethyl, C₁-C₄ alkoxycarbonyl, C₁-C₄ alkylthioethyl, pyridyl, thienyl, thenyl, tetrahydrofurfuryl, morpholino or phenyl which is unsubstituted or substituted with chlorine or C₁-C₄ alkyl; R² is hydrogen or C₁-C₄ alkyl; R³ is C₁-C₄ alkyl; X¹ is C₁-C₃ alkyl, C₁-C₃ alkoxy, or chlorine; X² is C₁ -C₃ alkyl or C₁-C₃ alkoxy; and Z is N or CH, which comprises: reacting 2-amino-4,6-di-substituted-1,3,5-triazine or -pyrimidine represented by the formula (IV) : 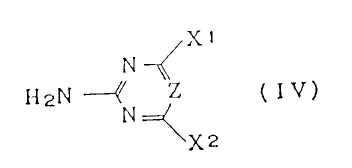 wherein X¹, X², and Z have the same meanings as defined above, with diphenyl carbonate to form a phenyl carbamate derivative represented by the formula (III): 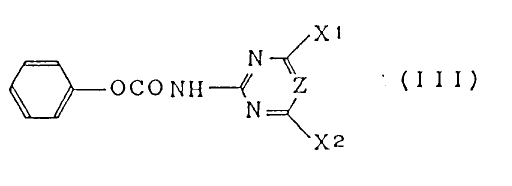 wherein X¹, X², and Z have the same meanings as defined above, and
further reacting the resulting product with 3-(substituted hydrazino)benzenesulfonamide derivative represented by the formula (II): 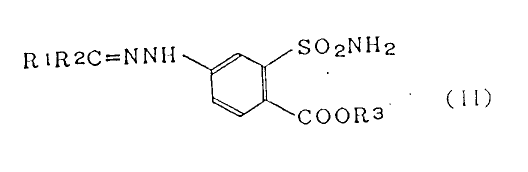 wherein R¹, R², and R³ have the same meanings as defined above, without any intermediate isolation.

5. A 3-(substituted hydrazino)benzenesulfonamide derivative represented by the formula (II): 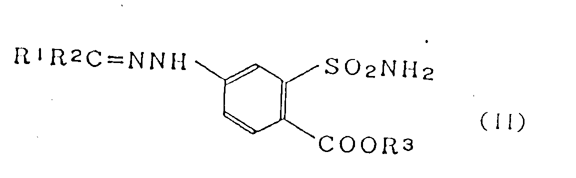 wherein R¹ is C₁-C₄ alkyl, monochloromethyl, C₃-C₄ alkenyl, C₁-C₄ alkoxymethyl, C₁-C₄ alkoxycarbonyl, C₁-C₄ alkylthioethyl, pyridyl, thienyl, thenyl, tetrahydrofurfuryl, morpholino or phenyl which is unsubstituted or substituted with chlorine or C₁-C₄ alkyl; and R2 is hydrogen or C₁-C₄ alkyl; R3 is C₁-C₄ alkyl; X¹ is C₁-C₃ alkyl, C₁-C₃ alkoxy, or chlorine.

6. A herbicidal composition containing a herbicidally effective amount of an N-substituted-3-(substituted hydrazino)benzenesulfonamide derivative as claimed in claim 1.

## Patentansprüche

1. N-substituierte-3-(substituierte Hydrazino)benzolsulfonamidderivate, dargestellt durch die folgende Formel (I): 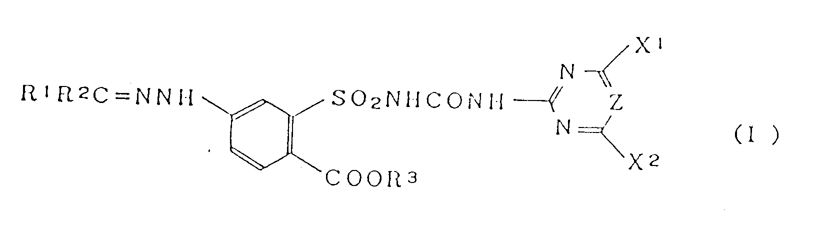 worin R¹ C₁-C₄-Alkyl, Monochlormethyl, C₃-C₄-Alkenyl, C₁-C₄-Alkoxymethyl, C₁-C₄-Alkoxycarbonyl, C₁-C₄-Alkylthioethyl, Pyridiyl, Thienyl, Thenyl, Tetrahydrofurfuryl, Morpholino oder Phenyl, welches unsubstituiert oder mit Chlor oder C₁-C₄-Alkyl substituiert ist, bedeutet, R² Wasserstoff oder C₁-C₄-Alkyl bedeutet, R³ C₁-C₄-Alkyl bedeutet, X¹ C₁-C₃-Alkyl, C₁-C₃-Alkoxy oder Chlor bedeutet, X₂ C₁-C₃-Alkyl oder C₁-C₃-Alkoxy bedeutet und Z N oder CH bedeutet.

2. N-substituierte-3-(substituierte Hydrazino)benzolsulfonamidderivate nach Anspruch 1, dadurch **gekennzeichnet,** daß Z in der allgemeinen Formel (I) N bedeutet.

3. N-substituierte-3-(substituierte Hydrazino)benzolsulfonamidderviate nach Anspruch 1, dadurch **gekennzeichnet,** daß Z in der allgemeinen Formel (I) CH bedeutet.

4. Verfahren zur Herstellung von N-substituierten-3-(substituierten Hydrazino)benzolsulfonamidderivaten, dargestellt durch die Formel (I): 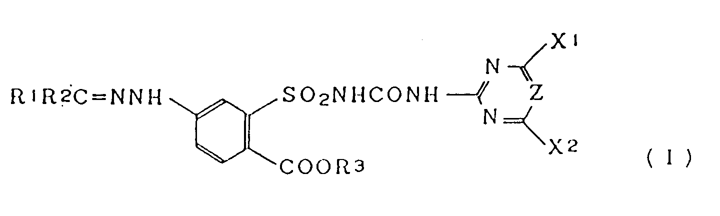 worin R¹ C₁-C₄-Alkyl, Monochlormethyl, C₃-C₄-Alkenyl, C₁-C₄-Alkoxymethyl, C₁-C₄-Alkoxycarbonyl, C₁-C₄-Alkylthioetyhl, Pyridyl, Thienyl, Thenyl, Tetrahydrofurfuryl, Morpholino oder Phenyl, welches unsubstituiert oder mit Chlor oder C₁-C₄-Alkyl substituiert ist, bedeutet, R² Wasserstoff oder C₁-C₄-Alkyl bedeutet, R³ C₁-C₄-Alkyl bedeutet, X¹ C₁-C₃-Alkyl, C₁-C₃-Alkoxy oder Chlor bedeutet, X₂ C₁-C₃-Alkyl oder C₁-C₃-Alkoxy bedeutet und Z N oder CH bedeutet, umfassend die Umsetzung eines 2-Amino-4,6-di-substituierten-1,3,5-triazins oder -pyrimidins, dargestellt durch die Formel (IV): 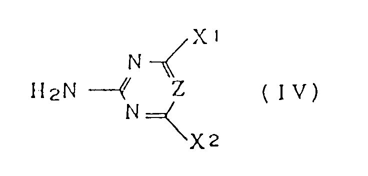 worin X¹, X² und Z die gleichen Bedeutungen wie oben definiert besitzen, mit Diphenylcarbonat unter Bildung eines Phenylcarbamatderivats, dargestellt durch die Formel (III): 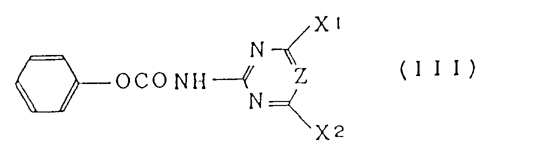 worin X¹, X² und Z die gleichen Bedeutungen wie oben gegeben besitzen, und weiter Umsetzung des entstehenden Produkts mit einem 3-(substituierten Hydrazino)benzol-sulfonamidderivat, dargestellt durch die Formel (II): 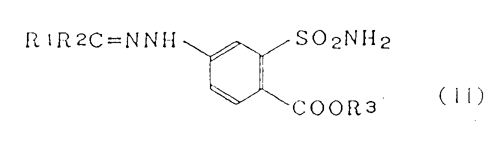 worin R¹, R² und R³ die gleichen Bedeutungen wie oben definiert besitzen, ohne Isolierung des Zwischenprodukts.

5. 3-(substituierte Hydrazino)benzolsulfonamidderivate, dargestellt durch die folgende Formel (II): 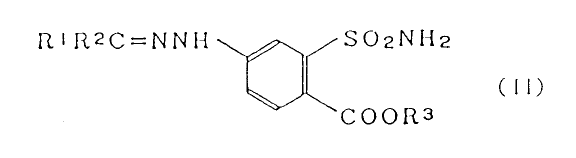 worin R¹ C₁-C₄-Alkyl, Monochlormethyl, C₃-C₄-Alkenyl, C₁-C₄-Alkoxymethyl, C₁-C₄-Alkoxycarbonyl, C₁-C₄-Alkylthioethyl, Pyridyl, Thienyl, Thenyl, Tetrahydrofurfuryl, Morpholino oder Phenyl, welches unsubstituiert oder mit Chlor oder C₁-C₄-Alkyl substituiert ist, bedeutet, und R² Wasserstoff oder C₁-C₄-Alkyl bedeutet, R³ C₁-C₄-Alkyl bedeutet, X¹ C₁-C₃-Alkyl, C₁-C₃-Alkoxy oder Chlor bedeutet.

6. Herbizides Mittel, enthaltend eine herbizid wirksame Menge eines N-substituierten-3-(substituierten Hydrazino)benzolsulfonamidderivats nach Anspruch 1.

## Revendications

1. Dérivé benzènesulfonamide N-substitué-3-(hydrazino-substitué) représente par la formule (I) suivante : 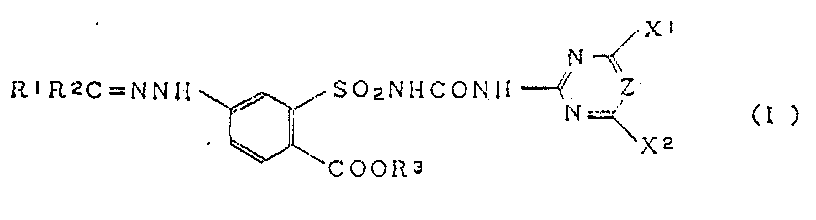 dans laquelle
- R¹ est un groupe alkyle (C₁-C₄), monochlorométhyle, alkényle (C₃-C₄), alcoxy(C₁-C₄)méthyle, alcoxy(C₁-C₄)carbonyle, alkyl(C₁-C₄)thioéthyle, pyridyle, thiényle, thényle, tétrahydrofurfuryle,morpholino ou phényle qui est substitué ou non substitué par le chlore ou un groupe alkyle(C₁-C₄);
- R² est l'hydrogène ou un groupe alkyle (C₁-C₄);
- R³ est un groupe alkyle (C₁-C₄)
- X¹ est un groupe alkyle (C₁-C₃), alcoxy (C₁-C₃), ou chloro;
- X² est un groupe alkyle (C₁-C₃) ou alcoxy (C₁-C₃); et
- Z représente l'azote ou un groupe CH.

2. Dérivé benzènesulfonamide N-substitué-3-(hydrazino-substitué) selon la revendication 1, dans lequel Z dans la formule générale (I) représente N.

3. Dérivé benzènesulfonamide N-substitué-3-(hydrazino-substitué) selon la revendication 1, dans lequel Z dans la formule générale (I) représente CH.

4. Procédé de production d'un dérivé benzènesulfonamide N-substitué-3-(hydrazino-substitué) représenté par la formule (I) : 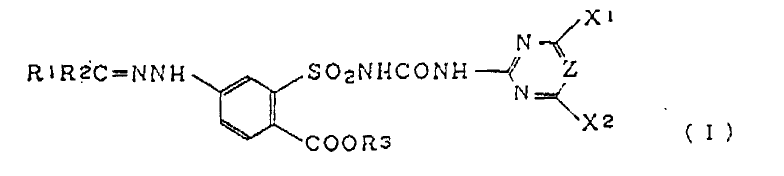 dans laquelle
- R¹ est un groupe alkyle (C₁-C₄), monochlorométhyle, alkényle (C₃-C₄), alcoxy(C₁-C₄)méthyle, alcoxy(C₁-C₄)carbonyle, alkyl(C₁-C₄)thioéthyle, pyridyle, thiényle, thényle,tétrahydrofurfuryle,morpholino ou phényle qui est substitué ou non substitué par le chlore ou un groupe alkyle(C₁-C₄);
- R² est l'hydrogène ou un groupe alkyle (C₁-C₄);
- R³ est un groupe alkyle (C₁-C₄);
- X¹ est un groupe alkyle (C₁-C₃), alcoxy (C₁-C₃), ou chloro;
- X² est un groupe alkyle (C₁-C₃) ou alcoxy (C₁-C₃); et
- Z représente l'azote ou un groupe CH,
qui comprend la réaction d'une 2-amino-4,6-disubstituée-1,3,5-triazine ou -pyrimidine représentée par la formule (IV) : 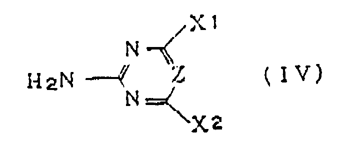 dans laquelle X¹, X² et Z sont tels que définis plus haut,
avec le carbonate de diphényle pour former un dérivé phényle carbamate représenté par la formule (III) : 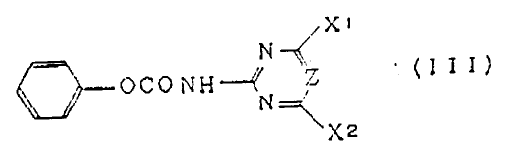 dans laquelle X¹, X² et Z sont tels que définis plus haut, et ensuite la réaction du produit ainsi obtenu, avec un dérivé benzènesulfonamide-3-(hydrazino-substitué) représenté par la formule (II) : 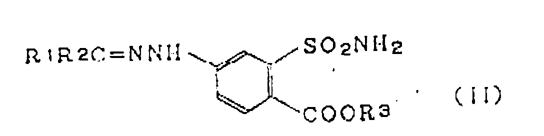 dans laquelle R¹, R² et R³ sont tels que définis plus haut, sans isolement d'aucun produit intermédiaire.

5. Dérivé benzènesulfonamide-3-(hydrazino-substitué) représenté par la formule (II) : 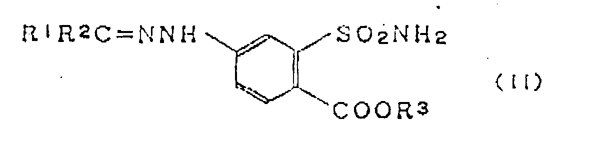 dans laquelle
- R¹ est un groupe alkyle (C₁-C₄), monochlorométhyle, alkényle (C₃-C₄), alcoxy(C₁-C₄)méthyle, alcoxy(C₁-C₄)carbonyle, alkyl(C₁-C₄)thioéthyle, pyridyle, thiényle,thényle, tétrahydrofurfuryle, morpholino ou phényle qui est substitué ou non substitué par le chlore ou un groupe alkyl(C₁-C₄);
- R² est l'hydrogène ou un groupe alkyle (C₁-C₄);
- R³ est un groupe alkyle (C₁-C₄) ;
- X¹ est un groupe alkyle (C₁-C₃), alcoxy (C₁-C₃), ou chloro;

6. Composition herbicide contenant une quantité efficace du point de vue herbicide, d'un dérivé benzènesulfonamide N-substitué-3-(hydrazino-substitué) selon la revendication 1.
